# EUROPEAN PATENT APPLICATION

(11) **EP 2 810 603 A1**
(43) Date of publication of application: **10.12.2014**
(21) Application number: 13744060.8
(22) Date of filing: 30.01.2013
(51) Int. Cl.: A61B 10/00, A61B 1/00

(54) **ILLUMINATION DEVICE FOR TUMOR DETECTION AND ILLUMINATION DEVICE FOR EXAMINATIONS**

(30) Priority: 30.01.2012 JP 2012017206
(71) Applicant: CCS Inc., Kyoto-City, Kyoto 602-8011 (JP)
(72) Inventor: ICHIKAWA, Akira, Kyoto-shi Kyoto 602-8011 (JP); SAITO, Mitsuru, Kyoto-shi Kyoto 602-8011 (JP); INOUE, Yoshihiro, Kyoto-shi Kyoto 602-8011 (JP)
(74) Representative: Müller - Hoffmann & Partner
(86) International application number: PCT/JP2013/051962
(87) International publication number: WO 2013/115209

(57) **Abstract**

The present invention is an illumination device for tumor detection 1, which improves the visibility of a tumor in which a fluorescent substance is accumulated, and emits excitation light for the fluorescent substance accumulated in the tumor, and the illumination device for tumor detection 1 is provided with: a light emitting diode 7 that emits light of which a wavelength range substantially coincides with an excitation wavelength range for the fluorescent substance; and a blocking part 8 that is provided on a light emission side of the light emitting diode 7, and blocks the same wavelength as at least a part of the excitation wavelength range for the fluorescent substance.

## Description

### Technical Field

The present invention relates to an illumination device for tumor detection, which when performing an operation to surgically excise a tumor having a fluorescent substance accumulating property, makes it possible to visually recognize a border of the tumor, or to an illumination device for examinations, which excites a fluorescent substance provided in an examination object.

### Background Art

In recent years, for example, in a brain tumor removal operation, a fluorescent substance having an accumulating property in a tumor has been administered to a patient and accumulated in a tumor, and then excited, for example, excitation light in the range from blue to violet has been irradiated onto a surgical field subjected to a craniotomy, and fluorescence having a predetermined color (e.g., a red color) emitted from the fluorescent substance by the irradiation has been used as a label to distinguish the tumor and normal tissue from each other. For example, in the case of using 5-aminolevulinic acid as the fluorescent substance, violet light serves as the excitation light, and the tumor site emits red fluorescence.

In addition, typically, the above-described tumor removal operation or a similar operation is performed with an external white illumination device being used to switch between light and darkness of the surgical field. Specifically, at the time of checking a tumor position, the white illumination device is turned off, or the white illumination device is turned in a different direction from a direction toward the surgical field to create a dark state, whereas at the time of inserting a scalpel, the surgical field is irradiated with white light by the external white illumination device to create a light state.

In particular, in the case of a tumor from which emitted fluorescence is weak, it is apparent that the tumor and normal tissue cannot be distinguished due to color mixture with the external white light. In order to prevent such a type of tumor from being missed, in medical sites, an external illumination device is strictly handled when switching between light and darkness of a surgical field, in order to prevent as much as possible unnecessary light such as white light from entering the surgical field when checking a tumor position. Also, some medical doctors may check a tumor position and insert a scalpel with keeping a dark state without using a white illumination device.

Meanwhile, as a light source part that emits such excitation light, for example, paragraphs such as paragraph [0025] of Patent Literature 1 describe a light source part adapted to attach an optical filter, which transmits a wavelength component of the excitation light, on a general white light source such as a halogen lamp, a metal halide lamp, or a light emitting diode. This is because, if white light is irradiated onto a tumor without an optical filter, fluorescence emitted from the tumor and the irradiated white light cannot be distinguished because the white light includes a wavelength range of the fluorescence, and consequently the tumor and its peripheral normal tissue cannot be distinguished from each other.

On the other hand, paragraph [0036] of Patent Literature 1 describes that as the light source part, a light emitting diode having characteristics where a wavelength range coincides with a wavelength range of the excitation light and is narrow can be applied. However, as far as the description shows, the necessity of the optical filter in the light source part is not described. Come to think of it, regarding such a light emitting diode of a single color type (a single peak wavelength type), emitted light thereof itself is monochromatic light that coincides in wavelength range with the excitation light, and therefore it can be said that there is no necessity at all to additionally attach an optical filter that transmits the excitation light wavelength range, in other words, an optical filter that cuts the same wavelength range as that of the fluorescence, and no attachment of an optical filter is the most reasonable design matter for one having ordinary skill in the art.

However, the present inventor has found that, in practice, when irradiating a surgical field with the emitted light of the light emitting diode of the single peak wavelength type as described above, which substantially coincides in wavelength range with the excitation light, not only a tumor emits red fluorescence, but its peripheral normal tissue part also sometimes appears to emit red light. Further, as the cause of this, the present inventor has found out that even the light emitting diode announced as emitting the light only having the wavelength component of the excitation light actually emits red light, which although weak in intensity, appears in the same color as that of the fluorescence, due to a cause such as a trace of unexpected component contained in a chip constituting the light emitting diode, or unexpected fluorescence from somewhere such as an reflective plate mounted with the chip.

The red light is extremely weak in intensity, and hardly matters for other purposes; however, for example, in a removal operation or examination of a brain tumor or the like, such extremely weak red light illuminates, for example, normal tissue of a brain in red, which may cause false recognition as red fluorescence emitted from the tumor to give rise to a big problem in a medical site where any error is not acceptable.

### Citation List

### Patent Literature

Patent Literature 1: JP-A2004-89533

### Summary of Invention

### Technical Problem

The present invention is made in consideration of the above-described problem first found by the present inventor, and a main desired object thereof is to provide an illumination device for tumor detection, which prevents a reduction in visibility of a tumor in which a fluorescence substance is accumulated. Solution to Problem

That is, the illumination device for tumor detection according to the present invention is one that emits excitation light for a fluorescent substance accumulated in a tumor, and provided with: a light emitting diode that emits light of which a wavelength range substantially coincides with an excitation wavelength range for the fluorescent substance; and a blocking part that is provided on a light emission side of the light emitting diode, and blocks the same wavelength as at least a part of the excitation wavelength range for the fluorescent substance.

If so, even in the case where the light emitting diode emits light (e.g., red light) having a wavelength range, which appears in the same color as that of fluorescence from the fluorescent substance, the blocking part blocks the red light, so that in the case of illuminating a surgical field, the fluorescent substance in the tumor is excited to emit the fluorescent, but normal cells around the tumor is not illuminated in a red color. For this reason, the visibility of the tumor can be improved to surely distinguish the tumor from the normal cells. Note that in the present invention, the red light refers to light having a wavelength range of, for example, 600 nm to 770 nm, and a more appropriate wavelength range is 620 nm to 650 nm.

Also, desirably, the illumination device for tumor detection is provided with switching means adapted to switch an illumination state. Note that the switching of the illumination state is performed along with, for example, switching between a tumor position checking state and a scalpel insertion state, and includes (1) switching between lighting and extinction of the light emitting diode, (2) in the case where the illumination device has another light source such as a white light source in addition to the light emitting diode, switching between lighting of the light emitting diode and lighting of another light source such as a white light source, (3) in conjunction with an external illumination device that is different from the illumination device for tumor detection, switching between lighting of the light emitting diode and lighting of the external illumination device, and other switching cases. The switching means may be configured to, for example, provide the illumination device for tumor detection with a switch such as a push button, and when the switch is operated, switch the illumination state; however, considering washing, disinfection, and the like, desirably, the switching means is configured to provide a vibration sensor inside the illumination device for tumor detection, and by giving vibration from outside, switch the illumination state.

In order to emit the excitation light toward the tumor with ensuring a necessary and sufficient light amount, desirably, the illumination device for tumor detection is provided with a plurality of light emitting diodes, and adapted to be further provided with a light condensing part that condenses light emitted from each of the light emitting diodes. The illumination device for tumor detection provided with the plurality of light emitting diode as described enables an illumination area to be expanded to improve the visibility of the tumor. Also, a load on an operation to operate the illumination device for tumor detection to change the illumination area can be reduced. In particular, in the case where the tumor is present on a bottom or lateral surface of the surgical field, the presence of the plurality of light emitting diodes is effective, and more effective in the case of illuminating a lateral surface of a deep part of the surgical field, where it is difficult to ensure a large irradiation angle. Further, because of the presence of the plurality of light emitting diodes, a shadowless effect can be obtained. This makes it possible to, in the case of illuminating the surgical field with the same positional relationship, make the light reach details as compared with light from a lamp having a smaller number of light emitting diodes, and improve the visibility of the fluorescence to perform an optimum operation. Specific configurations of the light condensing part include one in which the light condensing part is provided with: a lens that is provided paired with a corresponding one of the light emitting diodes, and condenses light emitted from the light emitting diode; and a lens holder that supports the lens.

In order to suppress the occurrence of flare in the light irradiated onto the tumor, desirably, the lens holder is integrally provided with an aperture that narrows the light emitted from the light emitting diode.

Specific embodiments for condensing lights respectively emitted from the plurality of light emitting diodes in a predetermined area include one in which a central axis of the lens is separated from a central axis of the aperture.

In order to suppress the blocking part from being damaged when washing or disinfecting the outside, and performance of the blocking part from being reduced, desirably, the blocking part is formed on a surface of the lens, which is not exposed outside.

In order to sufficiently excite the fluorescent substance accumulated in the tumor in such a configuration, desirably, a peak wavelength of the excitation light is set within a range of 401 nm or more. In particular, regarding the excitation light, the peak wavelength is desirably set within a range of 401 nm to 420 nm, and more desirably, set within a range of 401 nm to 409 nm. That is, if the excitation light has a wavelength range less than 401 nm, a harmful ultraviolet radiation component increases, and the excitation light damages, for example, normal tissue of a brain and therefore not suitable. On the other hand, a wavelength exceeding 409 nm reduces the ability to excite the fluorescent substance, and therefore it becomes difficult to distinguish the tumor. For these reasons, as the violet light, light having the above-described wavelength range of 401 nm to 409 nm is suitable.

Also, an illumination device for examinations according to the present invention is an illumination device for examinations, which emits excitation light for a fluorescent substance present in an examination object, and provided with: a light emitting diode that emits light of which a wavelength range substantially coincides with an excitation wavelength range for the fluorescent substance; and a blocking part that is provided on a light emission side of the light emitting diode, and blocks the same wavelength as at least a part of the excitation wavelength range for the fluorescent substance.

If so, even in the case where the light emitting diode emits light having a wavelength range, which appears in the same color as that of fluorescence from the fluorescent substance, the blocking part blocks the light, and therefore the visibility of the fluorescent substance present in the examination object can be improved.

### Advantageous Effects of Invention

According to the present invention configured as described, when performing an operation for removing a tumor, the visibility of the tumor can be improved.

### Brief Description of Drawings

[Fig. 1]

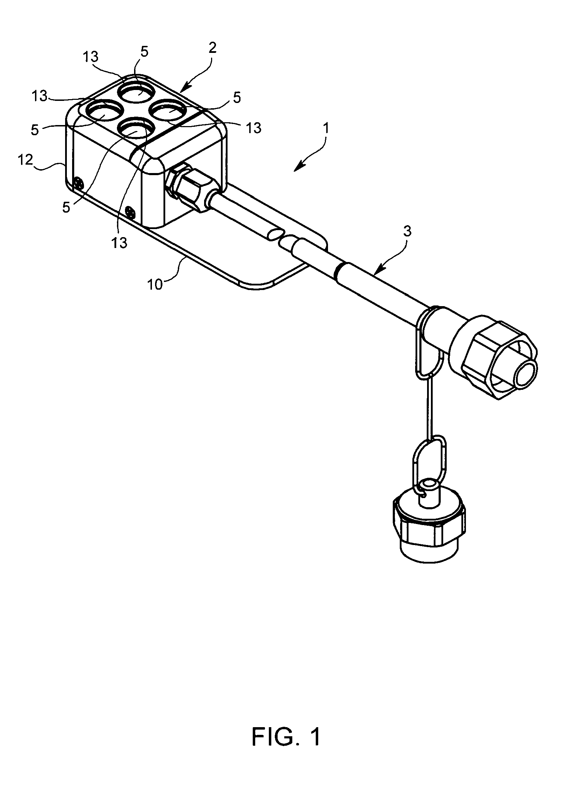

   Fig. 1 is a perspective view of an illumination device for tumor detection according to one embodiment of the present invention.
[Fig. 2]

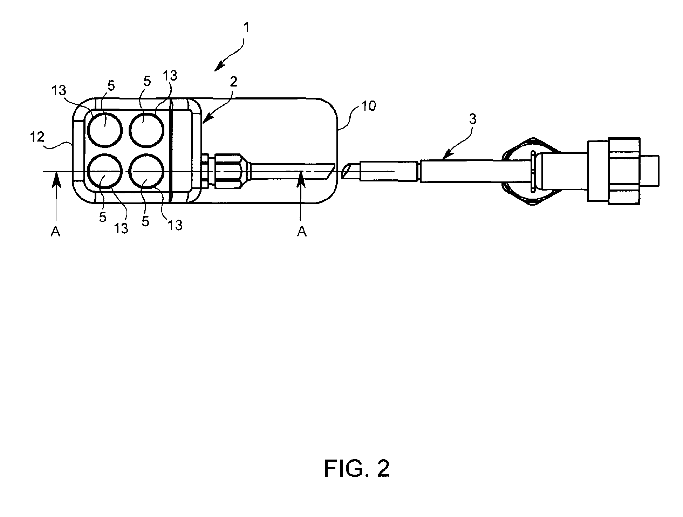

   Fig. 2 is a plan view according to the same embodiment.
[Fig. 3]

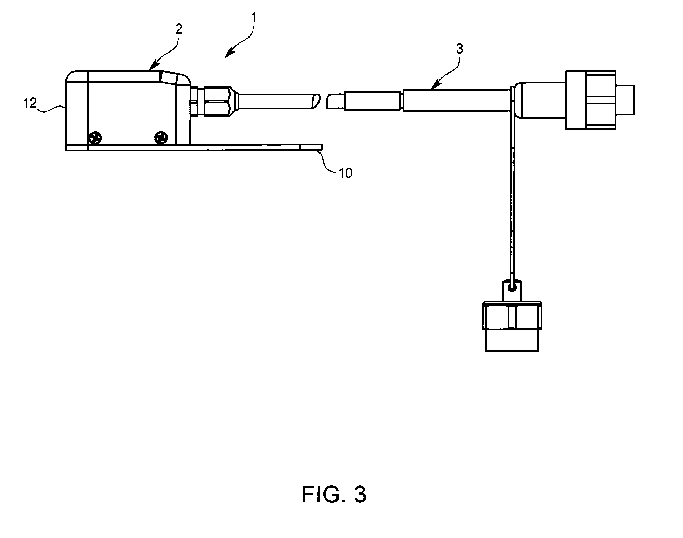

   Fig. 3 is a front view according to the same embodiment.
[Fig. 4]

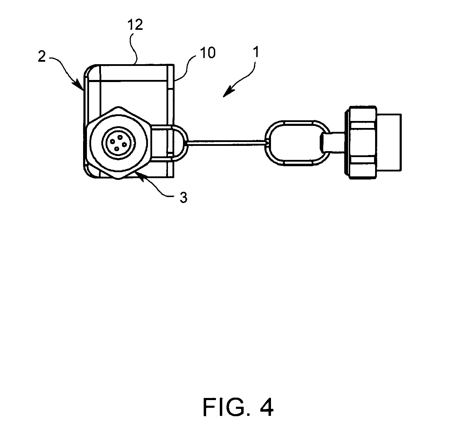

   Fig. 4 is a side view according to the same embodiment.
[Fig. 5]

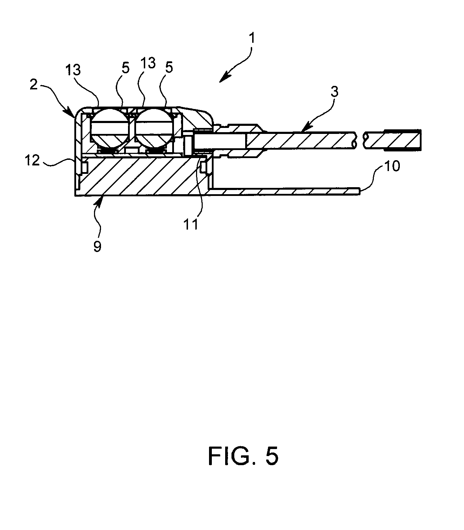

   Fig. 5 is a cross-sectional view along an A-A line according to the same embodiment.
[Fig. 6]

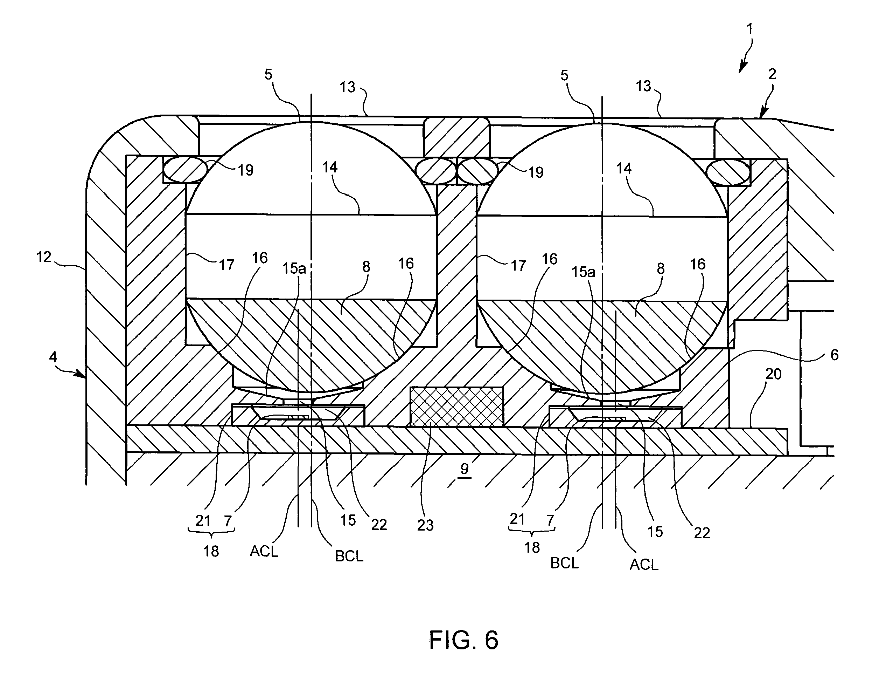

   Fig. 6 is a cross-sectional view illustrating an enlarged main part according to the same embodiment.
[Fig. 7]

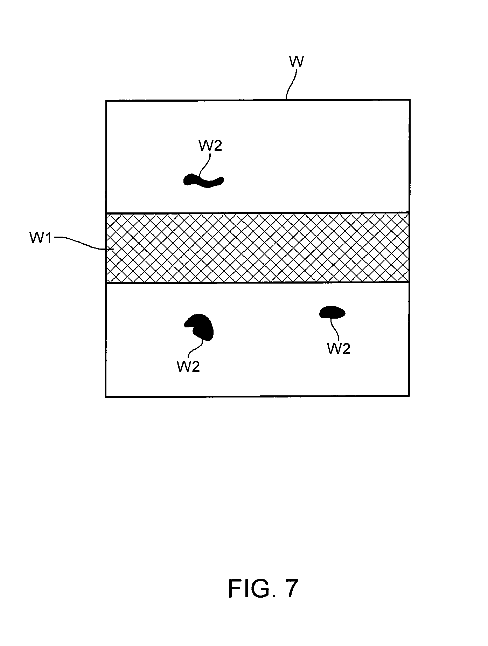

   Fig. 7 is a schematic diagram illustrating an examination object of an illumination device for examinations according to a variation. Reference Signs List

1 Illumination device for tumor detection
5 Lens
7 Light emitting diode
8 Filter
15 Aperture

### Description of Embodiments

In the following, one embodiment of the present invention is described with reference to drawings.

An illumination device for tumor detection (hereinafter also referred to as an illumination device) 1 according to the present embodiment is, as illustrated in Figs. 1 to 6, provided with a device main body 2, and a power cable 3 detachably attachable to the device main body 2. The device main body 2 is provided with: a casing 4; four lenses 5 that are incorporated in the casing 4; a lens holder 6 that supports the lenses 5; four light emitting diodes (hereinafter referred to as LEDs) 7; and filters 8 that are attached on the respective lenses 5. The illumination device 1 is one that is used to illuminate a surgical field when, for example, performing a craniotomy to excise a tumor in the head of a person with use of a fluorescent substance having an accumulating property in a tumor (in this embodiment, for example, 5-aminolevulinic acid (5-ALA) that emits red fluorescence). The illumination device 1 is used with repetitive washing and disinfection, and therefore water-proof and chemical-proof in structure.

The casing 4 is provided with: a base part 9; a gripping part 10 that is formed integrally with the base part 9; and a cover part 12 that is fixed to the base part 9 and forms a space incorporating the lenses 5, lens holder 6, light emitting diodes (hereinafter also referred to as the LEDs) 7, cable connecting part 11, and the like. The gripping part 10 is used to arrange the illumination device 1 in a desired location in such a way as to be clamped by a clamp or the like separately prepared in an operation room. The cover part 12 is, in a flat surface on the side opposite to the gripping part 10, provided with emission openings 13, which have an inside diameter smaller than a diameter of the lenses 5, correspondingly to positions of the lenses 5, and in a lateral surface in a longer direction, provided with an opening (not illustrated) for connecting the power cable 3 to the cable connecting part 11.

Each of the lenses 5 is held by the lens holder 6 that is positioned inside the cover part 12 by the cover part 12. The lens 5 in this embodiment is a so-called drum lens having a shape formed by integrally attaching semispherical plano-convex lenses respectively on the top and bottom of a cylinder. The lens 5 is provided with a cylindrically-shaped body part 14, which makes it easy to position and fix the lens 5 in the lens holder 6. In this embodiment, the lenses 5 are respectively formed with the filters 8, which serve as blocking parts that block red light, on surfaces of one convex lens-like end parts of the lenses 5.

Each of the filters 8 is formed on a surface of a corresponding one of the lenses 5, which faces to an aperture 15 provided for the lens 5, by evaporation, for example. That is, the filter 8 is one that is formed in a spherical part that is separated by the body part 14 of the lens 5 and not exposed from the cover part 12, and blocks the same wavelength as at least a part of an excitation wavelength range for the fluorescent substance from light emitted by the LED 7. Specifically, the filter 8 is one having characteristics of, when irradiating normal cells other than a tumor, blocking only light at a site of the irradiation, which is visible to a human as almost the same color as that of fluorescence, i.e., in this embodiment, only red light. Note that the filter 8 is a transparent thin film that is thin as compared with the size of the lens 5, and difficult to illustrate, so that in Fig. 6, the illustration of a cross section thereof is omitted, but instead, a formation area of the filter 8 is illustrated by hatching.

The lens holder 6 has the structure that individually supports and fixes the four lenses 5. Specifically, the lens holder 6 is provided with: lens seat parts 16 that support lower sides of the respective lenses 5; support wall parts 17 that support the cylindrically-shaped body parts 14 of the respective lenses 5; and the apertures 15 that let the lights from light emitting diode parts (hereinafter referred to as LED parts) 18 arranged with the LEDs 7 pass, respectively. Between lenses 5 supported by the lens holder 6 and the cover part 12, O-rings 19 are fitted for water-proofing and chemical-proofing.

Each of the lens seat parts 16 is an annular-shaped one, in which a seating surface in contact with a corresponding one of the lenses 5 is formed so as to have substantially the same curvature as a curvature of the lens 5. Further, the lens seat part 16 is a part of which a width continuously changes, and as illustrated in Fig. 6, in a cross section passing through the center of the lens 5, one of opposite widths is narrow, whereas the other width is wide as compared with the one width.

Each of the support wall parts 17 is subjected to reflection reducing treatment such as black resin or black coating so as not to reflect light emitted from the body part 14 of a corresponding one of the lenses 5, and thereby suppresses the occurrence of flare.

Each of the apertures 15 is provided integrally with the lens holder 6 on a lower side of a corresponding one of the lens seat parts 16. That is, the aperture 15 is provided in a circular plate part 15a continuous with the lens seat part 16. The circular plate part 15a has a cross-sectional shape of which a thickness on the lens seat part 16 side, i.e., a thickness on a base end side is large, and a thickness in an end part where the aperture 15 is formed, i.e., a thickness on a fore end side is smaller than the thickness on the base end side. As described, by forming the aperture 15 in the circular plate part 15a provided integrally with the lens holder 6, a distance between the lens 5 supported by the lens seat part 16 and the aperture 15 does not vary among products.

The size of the aperture 15 is desirably smaller in diameter than an opening diameter of an after-mentioned LED pedestal 21 of the LED part 18. In Fig. 6, the size of the aperture 15 is made larger in diameter than a diameter of the LED 7 in order to ensure output power; however, in the case where the output power from the LED 7 is sufficient, the size of the aperture 15 is further desirably smaller in diameter than the diameter of the LED in terms of being able to reduce the degree of unevenness of light in an irradiation spot. In such a case, for example, the size of the aperture 15 is desirably approximately 1.5 times the size of the LED 7. By setting the diameter of the aperture 15 to such a size, a necessary light amount can be ensured at the time of use, and also the occurrence of flare can be suppressed.

Aperture central axes ACL of the apertures 15 are respectively displaced from central axes BCL of corresponding ones of the lenses 5, and configured to condense the lights respectively having passed through the four lenses 5 at a predetermined position. In this case, given that the central axes BCL of the lenses 5 are set as reference positions, respectively, the aperture central axes ACL of the respective apertures 15 are displaced from the central axes BCL of the corresponding lenses 5 so as to be separated from each other, and in other words, arranged in locations receding from the central axes BCL of the corresponding lenses 5 toward the outside of the casing 4.

Each of the LED parts 18 is one provided on a board 20 fixed on the base part 9, and provided with: an LED pedestal 21 that is opened on the lens 5 side; and a corresponding one of the LEDs 7 provided on the LED pedestal 21. The LED part 18 is arranged in a concave portion 22 that is provided on a lower side of the aperture 15 of the lens holder 6. The LED pedestal 21 is formed so as to reflect the light emitted from the LED 7.

The LED 7 is arranged on the LED pedestal 21 so as to align the emission center thereof with the aperture central axis ACL of the aperture 15. The LED 7 has a single peak wavelength in its specifications, and is said to emit the light having the single peak wavelength that is, for example, 405 nm. The peak wavelength of the LED 7 is one that substantially coincides with the central wavelength of excitation light for exciting the fluorescent substance. "Substantially coincides" means that the peak wavelength and the central wavelength of the excitation light may be slightly shifted from each other; however, the amount of the shift is within a certain range, i.e., the amount of the shift falls within a range where the light that is emitted from the LED 7 and has a peak-shaped spectrum spreading over a wavelength range centering around the peak wavelength can excite the fluorescent substance to the extent that the fluorescence from the fluorescent substance is visible. An example where within a full width half maximum of the peak-shaped spectrum, the central wavelength of the excitation light is present can be cited. In addition, any of materials, structure, and the like of the LED 7 is not limited.

In the above configuration, in the case of making the LED 7 emit light, the light including light reflected by the LED seat part 16 is narrowed by the aperture 15, and then emitted outside the casing 4 through the lens 5. On the other hand, the lens 5 is formed integrally with the filter 8, and a trace of unexpected red color component included in the light emitted from the LED 7 is blocked by the filter 8, and therefore never passes through the lens 5. Accordingly, the light passing through the lens 5 includes the light reflected by the LED pedestal 21 and the light directly emitted from the LED 7 after the narrowing by the aperture 15, and a peak wavelength thereof is 405 nm in the violet range, which is a single peak. Further, the central axes BCL of the lenses 5 and the aperture central axes ACL of the corresponding apertures 15 deviate from each other, and therefore excitation lights having passed through the respective lenses 5 are condensed in a predetermined area distant from the cover part 12 by a predetermined distance.

Also, the illumination device 1 of the present embodiment has a vibration sensor 23 as vibration sensing means as illustrated in Fig. 6. The vibration sensor 23 is one that is intended to switch between lighting and extinction of the illumination device 1. Further, the illumination device 1 is configured to, in the case where the vibration sensor 23 senses vibration having the magnitude equal to or larger than a predetermined value, light the LEDs 7 in an extinction state, or extinguish the LEDs 7 in a lighting state. As described, by giving the illumination device 1 the vibration having the magnitude equal to or larger than the predetermined value, lighting/extinction of the LEDs 7 can be switched.

This makes it possible to simplify the switching between lighting and extinction of the illumination device 1, which is required be performed at the time of switching between scalpel insertion and a tumor position check, which is frequently performed during an operation, and consequently reduce an operator's stress at the time of the switching work.

Note that the switching using the vibration sensor 23 in the illumination device 1 is not limited to the switching between lighting and extinction of the LEDs 7, but may be configured to provide the illumination device 1 with a white light source, and switch between lighting of the LEDs 7 and lighting of the white light source. Alternatively, the switching may be configured to, in conjunction with an external white illumination device, when the LEDs 7 are lit, extinguish the external white illumination device, and when the LEDs 7 are extinguished, light the external white illumination device.

According to the illumination device 1 configured as described, even in the case where the LEDs 7 emit red light, the filters 8 blocks the red light, and therefore in the case of illuminating a surgical field, the fluorescent substance in a tumor is excited to emit the fluorescence, but normal cells around the tumor are not illuminated in a red color. For this reason, the visibility of the tumor can be improved to surely distinguish the tumor from the normal cells.

Also, the filters 8 are respectively formed on the surfaces of the lenses 5, and therefore the work for attaching the filters 8 can be eliminated. As a result, as compared with the case of setting up this sort of filter as an individual part, a man-hour for assembly can be reduced.

Further, each of the apertures 15, which narrows the light emitted from a corresponding one of the LED parts 18, is formed by the plate part 15a continuous with the lens seat part 16, and therefore a distance between the lens 5 and the aperture 15 does not vary among products, and therefore the yield can be improved.

Still further, without alignment, the lenses 5 can be fitted in the lens holder 6. Accordingly, the central axes BCL of the lenses 5 can be arranged at positions as set with respect to the aperture central axes ACL of the corresponding apertures 15, and therefore the lights emitted from the respective LEDs 7 can be condensed with the occurrence of flare being suppressed.

In addition, the filters 8 are respectively formed on the surfaces of the lenses 5 on an inner side of the cover part 12, and between the lenses 5 and the cover part 12, the O-rings 19 are arranged, so that at the time of washing and disinfecting the illumination device 1, the filters 8 can be prevented from being deteriorated due to contact with a washing or disinfectant solution.

Note that the present invention is not limited to the above-described embodiment.

Regarding the substantial peak wavelength of the light emitted by each of the LEDs 7 in the above-described embodiment, described are the four LEDs 7 all of which have the peak wavelength of 405 nm; however, without limitation to this, the present invention may use LEDs 7 in combination, which respectively emit lights having different wavelengths within the range of 401 nm to 409 nm, such as the wavelengths of 404 nm, 405 nm, 406 nm, and 407 nm.

In the above-described embodiment, the filters 8 are described as ones that are respectively formed on the surfaces of the lenses 5 by evaporation; however, although it is only necessary that the filters 8 are present in any locations as long as the filters 8 are provided in light paths along which the lights from the LEDs 7 reach an irradiation object, i.e., on light emission sides, locations that make it possible to surely prevent the filters 8 from being deteriorated due to the contact with a washing solution, a disinfectant solution, or the like are desirable. Accordingly, as in the above-described embodiment, in the case of forming the filters 8 respectively on the surfaces of the lenses, the filters 8 are formed on the surfaces located inside the casing 4. Also, regarding a way to attach the filters 8, in addition to forming the filters 8 respectively on the lenses 5 themselves as in the above-described embodiment, the filter 8 may be formed on translucent plates arranged as separate parts in spaces between the LEDs 7 and the corresponding lenses 5, respectively. Specifically, the filters 8 may be ones that are formed by evaporation on surfaces of the translucent plates fitted into the apertures 15, respectively.

The above-described embodiment uses the lenses 5; however, without limitation to this, the present invention may use lenses such as ball lenses, biconvex lenses, plano-convex lenses, semispherical lenses, or convex meniscus lenses.

Further, a way to suppress the occurrence of flare is not limited to the reflection reducing treatment such as black resin or black coating, but may be antireflection coating by vacuum evaporation, sputtering, or the like.

The present invention can be applied not only to a brain tumor but also to a tumor at another site to thereby obtain the same working effect.

In addition, the illumination device for tumor detection in the above-described embodiment, and eyeglasses worn by an operator or a microscope may be used together. In this case, the eyeglasses or the microscope is provided with optical filters that cut light having substantially the same color (e.g., light having a wavelength of 405 nm) as the color of the light irradiated from the illumination device for tumor detection. This makes it possible to clarify the difference between the fluorescence emitted from a surgical field and the light illuminating sites other than the surgical field to make it easy to see the fluorescence.

Also, as an illumination device for examinations according to the present invention, one that emits excitation light for a fluorescent substance present in an examination object is also possible. Specifically, the illumination device for examinations is provided with: a light emitting diode that, as illustrated in Fig. 7, emits light of which a wavelength range substantially coincides with, for example, an excitation wavelength range for a fluorescent substance contained in adhesives W1 and W2 provided in an examination object W; and a blocking part that is provided on a light emission side of the light emitting diode, and blocks the same wavelength as at least a part of the excitation wavelength range for the fluorescent substance. In Fig. 7, the adhesive W1 is an adhesive applied in a correct location, and the adhesive W2 is an adhesive applied in incorrect locations. By using the illumination device for examinations, the visibility of the adhesives W1 and W2 can be improved to surely determine whether or not the locations applied with the adhesives W1 and W2 are correct. That is, the adhesive W1 present in the incorrect locations can be surely grasped. In addition, a fluorescent substance present in the inspection object W is not limited to the fluorescent substance contained in the adhesive W.

Besides, the present invention is not limited to each of the above-described embodiments, but may be configured to appropriately combine parts or all of the above-described various configurations without departing from the scope thereof.

### Industrial Applicability

The present invention can provide the illumination device for tumor detection, which improves the visibility of a tumor in which a fluorescent substance is accumulated.

## Claims

1. An illumination device for tumor detection, the illumination device emitting excitation light for a fluorescent substance accumulated in a tumor, the illumination device comprising:
a light emitting diode that emits light of which a wavelength range substantially coincides with an excitation wavelength range for the fluorescent substance; and
a blocking part that is provided on a light emission side of the light emitting diode, and blocks a same wavelength as at least a part of the excitation wavelength range for the fluorescent substance.

2. The illumination device for tumor detection according to claim 1, wherein
the light emitted from the light emitting diode has a peak wavelength, and the peak wavelength is set within a range of 401 nm or more.

3. The illumination device for tumor detection according to claim 1, wherein
a color of fluorescence is red, and light blocked by the blocking part is red light.

4. The illumination device for tumor detection according to claim 1, wherein
the light emitted from the light emitting diode has a peak wavelength, and the peak wavelength is set within a range of 401 nm to 420 nm.

5. The illumination device for tumor detection according to claim 1, comprising
switching means adapted to switch an illumination state.

6. The illumination device for tumor detection according to claim 1, comprising
a plurality of light emitting diodes and a light condensing part that condenses light emitted from each of the light emitting diodes.

7. The illumination device for tumor detection according to claim 6, wherein
the light condensing part comprises:
a lens that is provided paired with a corresponding one of the light emitting diodes, and condenses light emitted from the light emitting diodes; and a lens holder that supports the lens.

8. The illumination device for tumor detection according to claim 7, wherein
the lens holder is integrally provided with an aperture that narrows the light emitted from the light emitting diode.

9. The illumination device for tumor detection according to claim 8, wherein
a central axis of the lens is separated from a central axis of the aperture.

10. The illumination device for tumor detection according to claim 7, wherein
the blocking part is formed on a surface of the lens, the surface not being exposed outside.

11. An illumination device for examinations, the illumination device emitting excitation light for a fluorescent substance present in an examination object, the illumination device comprising:
a light emitting diode that emits light of which a wavelength range substantially coincides with an excitation wavelength range for the fluorescent substance; and
a blocking part that is provided on a light emission side of the light emitting diode, and blocks a same wavelength as at least a part of the excitation wavelength range for the fluorescent substance.
